# EUROPEAN PATENT SPECIFICATION

(11) **EP 0 279 959 B1**
(45) Date of publication and mention of the grant of the patent: **11.08.1993**
(21) Application number: 87119370.2
(22) Date of filing: 30.12.1987
(51) Int. Cl.: A61M 25/00, A61M 29/02

(54) **Dilatation catheter with thin guide wire**
Dilatationskatheter mit dünnem Führungsdraht
Cathéter dilatateur avec fil de guidage mince

(30) Priority: 06.01.1987 US 646
(43) Date of publication of application: 31.08.1988
(73) Proprietor: ADVANCED CARDIOVASCULAR SYSTEMS, INC., Mountain View, CA 94039-7101 (US)
(72) Inventor: Machold, Timothy R., Moss Beach, California 94038 (US); Mueller, Richard L. Jr., Mountain View, California 94041 (US)
(74) Representative: Baillie, Iain Cameron

(56) References cited:
- EP-A- 0 231 601
- EP-A- 0 231 725
- GB-A- 2 172 205
- US-A- 4 538 622
- US-A- 4 616 653

## Description

### Technical Field

This invention pertains generally to medical applicances, and more particularly to a dilatation catheter and guide wire for use in coronary angioplasty.

### Background

In percutaneous transluminal coronary angioplasty, catheters are inserted into the cardiovascular system through the femoral or brachial arteries under local anesthesia. A preshaped guiding catheter is positioned in the coronary artery, and a dilatation catheter having a distensible balloon portion is advanced through this catheter into the branches of the coronary artery until the balloon portion traverses or crosses a stenotic lesion. The balloon portion is then inflated with a fluid to compress the atherosclerosis in a direction generally perpendicular to the wall of the artery, thereby dilating the lumen of the artery.

A guide wire is often employed to facilitate placement of the dilatation catheter beyond the distal end of the guiding catheter. The guide wire is inserted through the guiding catheter, and the dilatation catheter is advanced along the guide wire to the desired position in the vascular system.

It is generally desirable to make the guide wire as small in diameter and as flexible as possible in order to facilitate its insertion and guidance within the cardiovascular system. However, as a guide wire is made smaller, its ability to transmit torsional and axial forces is reduced, and the minimum usable size of guide wires has been limited by these considerations and by the fragility of smaller guide wires. Heretofore, the smallest guide wires utilized in coronary angioplasty have been on the order of .3-. 45 mm (.012-. 018 inch) in diameter.

In addition, catheters and guide wires heretofore provided have been designed to function independently of each other in providing torsional and axial rigidity. This permits the guide wires and catheters to be exchanged and utilized in a number of different combinations during a given angioplasty procedure.

Some prior art inventions are disclosed in the following publications. EP-A-0231601 discloses a catheter body and guide wire. The guide wire is sized to allow fluid flow between it and the inside surface of the catheter body.

U.S. Patent No. 4,616,653 related to a steerable fixed guide wire dilatation catheter. It includes a nonremovable guide wire and the inner lumen of the dilatation catheter and of the guide wire are small.

It is in general, a goal of the invention to provide a new and improved catheter and guide wire for use in coronary angioplasty.

Another goal of the invention is to provide a catheter and guide wire wherein the guide wire is smaller than guide wires heretofore employed in coronary angioplasty.

These and other goals are achieved in accordance with the invention by providing a catheter having a luminal opening in which a guide wire of relatively small diameter is received. The luminal opening is only slightly greater in diameter than the guide wire, and the luminal wall in close proximity to the guide wire supports the relatively thin guide wire against buckling and enhances the transmission of torsional and axial forces through the guide wire. Toward the distal end of the catheter, the luminal opening is lined with a lubricous material such as Teflon, and toward the proximal end the luminal opening is formed in a tubular member of stiffer material such as stainless steel. The distal end portion of the guide wire has a plurality of steps of progressively smaller diameter, with gentle tapers between the steps.

### Summary of the Invention

A dilatation catheter made in accordance with the invention has an outer tubular member with a distensible portion forming an inflatable balloon toward the distal end thereof, with an annular passageway for inflating the balloon being formed between the inner and outer tubular members. The outer tubular member is relatively flexible, and a relatively stiff tubular member is positioned within the proximal end portion of the outer tubular member. A two-arm adapter is connected to the proximal end of the catheter with a first port having a passageway of substantially the same diameter as the luminal opening aligned with the luminal opening in a second port in fluid communication with the annular passageway through which the balloon is inflated.

In summary, disclosed herein is an apparatus for use in angioplasty comprising an elongated guide wire of relatively small diameter, and a catheter having an elongated tubular member having a luminal opening of only slightly greater diameter than the diameter of the guide wire which extends therethrough, said apparatus being characterized in that: said guide wire is supported by the wall of the opening to enhance the transmission of torsional and axial forces through the wire and to prevent buckling of the wire, said elongated tubular member being fabricated of a lubricous material, said guide wire having an outer diameter less than .0254 cm, said luminal opening having a diameter which is not more than .00762 cm greater than said guide wire.

### Brief Description of the Drawings

FIGURE 1 is a side elevational view, partly broken away, of one embodiment of a dilatation catheter and guide wire according to the invention.

FIGURE 2 is an enlarged cross-sectional view taken along line 2-2 in Figure 1.

FIGURE 3 is an enlarged cross-sectional view taken along line 3-3 in Figure 1.

FIGURE 4 is an enlarged cross-sectional view taken along line 4-4 in Figure 1.

FIGURE 5 is an enlarged side elevational view of the guide wire in the embodiment of Figure 1.

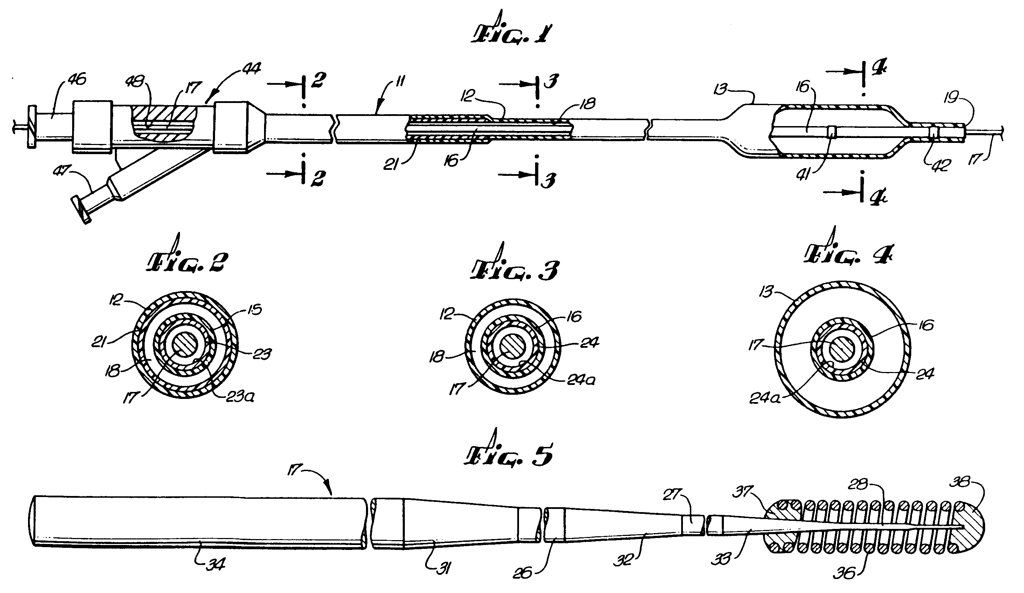

### Detailed Description of the Invention

As illustrated in the drawings, the catheter 11 has an axially extending outer tubular member 12 with a distensible portion forming an inflatable balloon 13 near the distal end thereof. An inner tubular member of shaft 16 extends coaxially within the outer tubular member, with a guide wire 17 extending through a luminal opening in the inner tubular member. The distal end portions of the tubular members are joined together by suitable means such as heat sealing to close the distal end of the balloon, and an annular passageway 18 is formed between the proximal end portions of the tubular members for inflation and deflation of the balloon. A vent opening 19 large enough to pass gas molecules but small enough to block the passage of liquid molecules communicates with the distal end of the balloon for venting trapped gas from the balloon as it is filled.

Outer tubular member 12 is fabricated of a relatively flexible material such as a low density polyethylene. The proximal end portion of this tubular member is stiffened by an inner layer 21 of a stiffer material such as a higher density polyethylene. The outer tubular member has a length on the order of 125-150 cm, and the stiffer tubular member or liner extends approximately 80-100 cm down the outer tubular member, tapering to a wall thickness on the order of .025 mm (.001 inch) over the last 20 cm, or so, of its length. The two tubular members are affixed together by heat sealing or other suitable means to form a laminated structure. The catheter is typically inserted into the body through a guiding catheter having a relatively straight proximal end portion and a curved distal end portion which is received within the aortic arch. The laminated structure provides maximum force transmission through the outer shaft of the dilatation catheter in the relatively straight portion of the guiding catheter, with a tapered stiff shaft over the aortic arch bend and maximum flexibility beyond the guiding catheter for better tracking over a guide wire.

Inner tubular member 16 is also fabricated of a relatively flexible material such as a low density polyethylene, and it has a tubular liner 23 of relatively stiff material such as stainless steel within its proximal end portion. The distal end portion of the tubular member 16 has a tubular liner 24 of lubricious material such as polytetraethylene which is sold under the United States registered trademark, Teflon, by Dupont (hereinafter "Teflon").

In one presently preferred embodiment, the catheter has an overall length of 130 cm, with the stainless steel tube extending for the first 30 cm of the inner tubular member and the Teflon tube extending for the last 100 cm of the tubular member. In this embodiment, the stainless steel tube has a luminal opening 23a of .33 mm (.013 inch) and an outside diameter of .56 mm (.022 inch), and the Teflon tube has a luminal opening 24a of .33 mm (.013 inch) and a wall thickness of .08 mm (.003 inch). Tubular member 16 is heat shrunk over the stainless steel tube and the Teflon tube, with the luminal openings in the stainless steel tube and the Teflon tube aligned axially with each other to form a smooth, continuous passageway for guide wire 17. The Teflon liner extends beyond the distal end of the balloon to the tip of the catheter and makes the distal end portion of the catheter resistant to bending and crumpling when initially piercing a tight stenosis.

The stainless steel tube in the proximal end portion of inner tubular member 16 greatly increases the ability of the catheter to track over the guide wire by transferring force from the unsupported portion of the catheter shaft outside the guiding catheter down the shaft to the catheter tip for better tracking on the wire and better stenosis crossing ability. Because of the relatively small luminal openings in the stainless steel tube and the Teflon tube, the walls of these openings are in close proximity to the guide wire, and they support the relatively thin guide wire to increase the transmission of both torsional and axial forces through the guide wire and prevent buckling of the wire. In addition, the Teflon liner reduces friction between the guide wire and the luminal wall. The Teflon material has the additional advantage of providing increased lubricity with increases in pressure. Thus, when the guide wire presses against the Teflon liner, the lubricity of the material increases, and the chance of the guide wire binding within the catheter is greatly reduced. Further, as the catheter is advanced across a lesion, the tight interface between the catheter and the guide wire and the lubricious quality of the system allow the guide wire to better support the catheter. Thus, most of the axial force goes into crossing the lesion and not into catheter buckling or crumpling.

Guide wire 17 is a relatively thin stainless steel wire having a diameter of only 0.25 mm (.010 inch) at its proximal end in one presently preferred embodiment. In this embodiment, the wire has a 0.013 mm (.0005 inch) thick Teflon coating, giving the wire an overall diameter of 0.28 mm (.011 inch). The distal end portion of the guide wire has three sections 26-28 of progressively smaller diameter, with gentle tapers 31-33 between the sections. In the embodiment illustrated, the guide wire has an overall length of 175 cm, and proximal end portion 34 has a length of about 140 cm and a diameter of 0.25 mm (.010 inch), section 26 has a length of about 7 cm and a diameter of 0.2 mm (.008 inch), section 27 has a length of about 18 cm and a diameter of 0.15 mm (.006 (.006 inch), and section 28 has a length of about 1 cm and a diameter of 0.1 mm (.004 inch), with the last .75 cm at the distal end of section 28 being flattened to a thickness of 0.02 mm (.0008 inch). Each of the tapered sections 31-33 is about 3 cm in length and provides a reduction in diameter of only 0.05 mm (.002 inch) over this length.

A helical coil 36 having a length of about 2-3 cm and an outside diameter of about 0.25 mm (.010 inch) is positioned coaxially about the distal end portion of the guide wire shaft and is affixed to the shaft by suitable means such as solder beads 37, 38. In one presently preferred embodiment, the helical coil is fabricated of a platinum wire having a diameter on the order of 0.06 mm (.0025 inch) to make the tip of the coil visible to a fluoroscope.

Radiopaque markers 41, 42 are mounted on inner tubular member 16 at the mid-point of the balloon and at the distal end of the catheter so that the position of the catheter can be observed with a fluoroscope. These markers can, for example, comprise bands of gold mounted on the tubular member.

A two-arm adapter 44 is provided at the proximal end of the catheter. This adapter has a central port 46 which communicates with the luminal openings 23a, 24a through which the guide wire extends, and a side port 47 in fluid communication with the annular passageway 18 through which the balloon is inflated. Central port 46 has an axial passageway 48 of the same diameter as luminal openings 23a, 24a.

In use, catheter 11 is inserted into the cardiovascular system through a guiding catheter (not shown). Guide wire 17 is inserted through luminal openings 23a, 24a and advanced beyond the distal end of catheter 11 for manipulation into the desired portion of the cardiovascular system. Catheter 11 is then advanced along the guide wire until balloon 13 traverses or crosses the lesion to be treated. The balloon is then inflated with pressurized fluid introduced through adapter port 47 and passageway 18.

Since the luminal opening of the catheter is only slightly greater 0.05 mm-0.075 mm (.002-.003 inch) than the diameter of the guide wire, the side wall of the luminal opening is in close proximity to the guide wire. The catheter is thus able to support the relatively flexible guide wire to enhance the transmission of both torsional and axial forces through the wire and to prevent buckling of the wire within the luminal opening. The lubricity of the Teflon liner reduces friction between the guide wire and the catheter and prevents binding of the guide wire within the catheter. As the catheter is advanced across a lesion, the tight interface between the catheter and the guide wire and the lubricious quality of the system allow the guide wire to better support the catheter. Thus, most of the axial force goes into crossing the lesion and not into catheter buckling or crumpling.

Although the invention has been described with specific reference to a 0.25 mm (.010 inch) guide wire a 0.013 mm (.0005 inch) Teflon coating, it can be utilized with other guide wires of larger or smaller diameter, with corresponding increases or decreases in the size of the luminal opening in the catheter in order to maintain the supporting relationship between the guide wire and the catheter.

It is apparent from the foregoing that a new and improved dilatation catheter and guide wire have been provided. While only certain presently preferred embodiments have been described in detail, as will be apparent to those familiar with the art, certain changes and modifications can be made without departing from the scope of the invention as defined by the following claims.

## Claims

1. An apparatus for use in angioplasty comprising an elongated guide wire (17) of relatively small diameter, and a catheter (11) having an elongate tubular shaft (16) having a luminal opening of only slightly greater diameter than the diameter of the guide wire (17) which extends therethrough, said apparatus being characterized in that: said guide wire (17) is supported by the wall of the opening to enhance the transmission of torsional and axial forces through the wire and to prevent buckling of the wire (17), said elongate tubular shaft being fabricated of a lubricous material, said guide wire (17) having an outer diameter less than .0254 cm (0.010 inch), said luminal opening having a diameter which is not more than .00762 cm (0.003 inch) greater than said guide wire (17).

2. The apparatus of claim 1 further characterized in that said guide wire (17) is removable and has an outer diameter of about 0.015 cm (0.006 inch) to about 0.025 cm (0.01 inch).

3. The apparatus of claims 1 or 2 further characterized in that the tubular shaft (16) is at least partially fabricated from polytetrafluoroethylene.

4. The apparatus of claim 1 further characterized in that the guide wire (17) has a diameter of about 0.28 mm (0.011 inches), and the inner lumen has an inner diameter of about 0.33 mm (0.013 inches).

5. The apparatus of claims 1, 2, 3 or 4 further characterized in that the catheter (11) has an inflatable balloon (13) positioned coaxially about the tubular shaft (16) toward the distal end of the catheter (11).

6. The apparatus of claim 5 wherein the tubular member (16) extends beyond the distal end of the balloon (13).

7. The apparatus of any one of claims 1 through 6 further characterized by a tubular liner (24) fabricated of lubricous material and extending coaxially within the distal end portion of said tubular shaft (16).

8. The apparatus of claim 5 further characterized by said catheter (11) including an outer tubular member (12) which includes said inflatable balloon (13); said tubular shaft (16) extending coaxially within said outer tubular member (12) with a passageway being formed between the outer wall of said shaft (16) and the inner wall of the outer tubular member (12) for inflation of the balloon; a polytetrafluoroethylene liner (24) having a luminal opening of relatively small diameter and extending axially within said tubular shaft (16), said guide wire (17) extending axially within said luminal opening in its extension in said tubular shaft (16).

9. The apparatus of claim 8 characterized in that said liner (24) extends beyond the distal end of the balloon (13).

10. The apparatus of claims 8 or 9 further characterized by a tube (23) which is stiffer than the liner (24), said tube (23) being disposed within the proximal end portion of the inner tubular shaft (16) and having a luminal opening of substantially the same diameter as the luminal opening of said liner (24).

11. The apparatus of claim 10 further characterized in that the tube (23) is fabricated of stainless steel.

12. The apparatus of any of claims 8 through 11 further characterized by a two-arm adapter (44) connected to the proximal end of the catheter (11) and having a first port (46) in fluid communication with the luminal opening in the inner tubular member and a second port (47) which is in fluid communication with the annular passage (18), the first port (46) having an axial passageway (48) of substantially the same diameter as the luminal opening defined by the liner (24) and tube (23), said axial passageway being in alignment with said openings.

13. The apparatus of any one of the preceding claims characterized in that the distal end portion of the guide wire (17) has a plurality of sections (26-28) of progressively smaller diameter with gradual tapers (31-33) between the sections.

14. The apparatus of claim 13 characterized by a helical coil (36) at the distal end of the guide wire (17), said helical coil (36) having an outer diameter no greater than the diameter of the luminal opening in said liner (24).

15. The apparatus of any one of claims 8 through 14 characterized by a relatively stiff tubular member (21) situated coaxially within the proximal end portion of the outer tubular member (12).

16. The apparatus of claim 15 characterized in that the distal end portion of the tubular member (21) tapers to a thinner wall diameter toward its distal end.

17. The apparatus of any one of claims 8 through 16 further characterized in that said outer tubular member (12) is relatively flexible and extends the full length of the balloon dilatation catheter.

18. The balloon dilatation catheter assembly of any one of claims 10 through 17 characterized in that the luminal openings in said liner (24) and said tube (23) have a diameter of .33 mm (.013 inches).

19. The apparatus of any one of claims 4 through 18 further characterized in that said catheter body (11) having said inflatable balloon (13) has a first inner lumen which is in fluid communication with the interior of the inflatable balloon (13) and a source of inflation fluid, and means to seal the distal end of the balloon (13) to prevent loss of inflation fluid from the interior thereof.

## Patentansprüche

1. Eine Vorrichtung zur Verwendung in der Angioplastik, wobei die Vorrichtung einen länglichen Führungsdraht (17) mit verhältnismäßig geringem Durchmesser aufweist, sowie einen Katheter (11) mit einem länglichen Röhrenelement (16), welches eine Luminalöffnung aufweist, deren Durchmesser nur geringfügig größer ist als der Durchmesser des sich durch die Luminalöffnung erstreckenden Führungsdrahtes (17), wobei die genannte Vorrichtung dadurch gekennzeichnet ist, daß: der genannte Führungsdraht (17) durch die Wand der Öffnung gestützt wird, um so die Übertragung von Dreh- und Längskräften durch den Draht zu verbessern, und um ein Knicken des Drahtes (17) zu verhindern; das genannte längliche Röhrenelement aus einem gleitenden Material hergestellt ist; der genannte Führungsdraht (17) einen Außendurchmesser von weniger als 0,0254 cm (0,010 Inch) aufweist; die genannte Luminalöffnung einen Durchmesser aufweist, der um nicht mehr als 0,00762 cm (0,003 Inch) größer ist als der Durchmesser des genannten Führungsdrahts (17).

2. Vorrichtung nach Anspruch 1, ferner dadurch gekennzeichnet, daß der genannte Führungsdraht (17) entfernbar ist und einen Außendurchmesser von etwa 0,015 cm (0,006 Inch) bis etwa 0,025 cm (0,01 Inch) aufweist.

3. Vorrichtung nach Anspruch 1 oder 2, ferner dadurch gekennzeichnet, daß das genannte Röhrenelement (16) wenigstens zum Teil aus Polytetrafluorethylen hergestellt ist.

4. Vorrichtung nach Anspruch 1, ferner dadurch gekennzeichnet, daß der Führungsdraht (17) einen Durchmesser von etwa 0,28 mm (0,011 Inch) aufweist, und daß der innere Lumen einen Innendurchmesser von etwa 0,33 mm (0,013 Inch) aufweist.

5. Vorrichtung gemäß den Ansprüchen 1, 2, 3 oder 4, ferner dadurch gekennzeichnet, daß der Katheter (11) einen auffüllbaren Ballon (13) aufweist, der koaxial um das Röhrenelement (16) in Richtung des fernen Endes des Katheters (11) positioniert ist.

6. Vorrichtung nach Anspruch 5, dadurch gekennzeichnet, daß sich das Röhrenelement (16) über das ferne Ende des Ballons (13) hinaus erstreckt.

7. Vorrichtung gemäß einem der Ansprüche 1 bis 6, ferner gekennzeichnet durch eine Röhreneinlage (24), die aus einem gleitenden Material hergestellt ist, und die sich koaxial in dem fernen Endteil des genannten inneren Röhrenelements (16) erstreckt.

8. Vorrichtung nach Anspruch 5, ferner dadurch gekennzeichnet, daß der genannte Katheter (11) ein äußeres Röhrenelement (12) aufweist, welches den genannten auffüllbaren Ballon (13) umfaßt; daß sich das genannte Röhrenelement (16) koaxial in dem genannten äußeren Röhrenelement (12) erstreckt, wobei ein Kanal zwischen der äußeren Wand des genannten Röhrenelements (16) und der inneren Wand des äußeren Röhrenelements (12) gebildet wird, und zwar zum Auffüllen des Ballons; gekennzeichnet durch eine Polytetrafluorethylen-Einlage (24) mit einer Luminalöffnung mit verhältnismäßig geringem Durchmesser, wobei sich die Einlage axial in dem genannten Röhrenelement (16) erstreckt, dadurch gekennzeichnet, daß sich der genannte Führungsdraht (17) axial in der genannten Luminalöffnung, in deren Ausweitung in dem genannten Röhrenelement (16) erstreckt.

9. Vorrichtung nach Anspruch 8, dadurch gekennzeichnet, daß sich die genannte Einlage (24) über das ferne Ende des Ballons (13) hinaus erstreckt.

10. Vorrichtung nach Anspruch 8 oder 9, ferner gekennzeichnet durch ein Rohr (23), welches steifer ist als die Einlage (24), wobei sich das genannte Rohr (23) innerhalb des nahen Endteils des inneren Röhrenelements (16) befindet, und wobei es eine Luminalöffnung aufweist, die im wesentlichen den gleichen Durchmesser aufweist wie die Luminalöffnung der genannten Einlage (24).

11. Vorrichtung nach Anspruch 10, ferner dadurch gekennzeichnet, daß das Rohr (23) aus rostfreiem Stahl hergestellt ist.

12. Vorrichtung gemäß einem der Ansprüche 8 bis 11, ferner gekennzeichnet durch ein doppelarmiges Paßstück (44), welches mit dem nahen Ende des Katheters (11) verbunden ist, und wobei das Paßstück einen ersten Anschluß (46) aufweist, der in strömender Verbindung mit der Luminalöffnung in dem inneren Röhrenelement steht, und wobei das Paßstück einen zweiten Anschluß (47) aufweist, der in strömender Verbindung mit dem ringförmigen Kanal (18) steht, dadurch gekennzeichnet, daß der erste Anschluß (46) einen axialen Kanal (48) umfaßt, der im wesentlichen den gleichen Durchmesser aufweist wie die durch die Einlage (24) und das Rohr (23) definierte Luminalöffnung, wobei sich der genannte axiale Kanal in Ausrichtung mit den Öffnungen befindet.

13. Vorrichtung gemäß einem der vorstehenden Ansprüche, dadurch gekennzeichnet, daß der ferne Endteil des Führungsdrahts (17) eine Mehrzahl von Teilstücken (26-28) mit einem progressiv kleineren Durchmesser aufweist, und zwar mit stufenweis konischen Stücken (31-33) zwischen den Teilstücken.

14. Vorrichtung nach Anspruch 13, gekennzeichnet durch eine gewundene Spule (36) an dem fernen Ende des Führungsdrahts (17), wobei die genannte gewundene Spule (36) einen Außendurchmesser aufweist, der nicht größer ist als der Durchmesser der Luminalöffnung in der genannten Einlage (24).

15. Vorrichtung gemäß einem der Ansprüche 8 bis 14, gekennzeichnet durch ein verhältnismäßig steifes Röhrenelement (21), welches sich koaxial innerhalb des nahen Endteils des äußeren Röhrenelements (12) befindet.

16. Vorrichtung nach Anspruch 15, dadurch gekennzeichnet, daß der ferne Endteil des Röhrenelements (21) in Richtung seines fernen Endes zu einer dünneren Wand konisch zuläuft.

17. Vorrichtung gemäß einem der Ansprüche 8 bis 16, ferner dadurch gekennzeichnet, daß das genannte äußere Röhrenelement (12) verhältnismäßig elastisch ist und sich über die gesamte Länge des Ballon-Dilatationskatheters erstreckt.

18. Der Ballon-Dilatationskatheter-Zusammenbau gemäß einem der Ansprüche 10 bis 17, dadurch gekennzeichnet, daß die Luminalöffnungen in der genannten Einlage (24) und dem genannten Rohr (23) einen Durchmesser von 0,33 mm (0,013 Inch) aufweisen.

19. Vorrichtung gemäß einem der Ansprüche 4 bis 18, ferner dadurch gekennzeichnet, daß das genannte Katheterstück (11), welches den genannten auffüllbaren Ballon (13) umfaßt, einen ersten inneren Lumen aufweist, der in strömender Verbindung mit dem Inneren des auffüllbaren Ballons (13) steht, sowie eine Füllflüssigkeitsquelle und eine Einrichtung zur Abdichtung des fernen Endes des Ballons (13), um einen Verlust von Füllflüssigkeit aus dem Inneren des Ballons zu verhindern.

## Revendications

1. Appareil à utiliser en angioplastie, comprenant un fil de guidage allongé (17) de diamètre relativement faible et un cathéter (11) muni d'une tige tubulaire allongé (16) ayant une ouverture luminale de diamètre uniquement légèrement supérieur au diamètre du fil de guidage (17) qui la traverse, ledit appareil étant caractérisé en ce que : ledit fil de guidage (17) est porté par la paroi de l'ouverture pour amplifier la transmission des forces de torsion et des forces axiales par le fil et pour prévenir le bouclage du fil (17), ladite tige tubulaire allongé étant en un matériau antifriction, ledit fil de guidage (17) ayant un diamètre externe inférieur à 0,0254 cm (0,010 pouce), ladite ouverture luminale ayant un diamètre supérieur au maximum de 0,00762 cm (0,003 pouce) à celui dudit fil de guidage (17).

2. Appareil selon la revendication 1, caractérisé en outre en ce que ledit fil de guidage (17) est amovible et possède un diamètre externe compris entre environ 0,015 cm (0,006 pouce) et environ 0,025 cm (0,01 pouce).

3. Appareil selon la revendication 1 ou 2, caractérisé en outre en ce que la tige tubulaire (16) est au moins partiellement en polytétrafluoroéthylène.

4. Appareil selon la revendication 1, caractérisé en outre en ce que le fil de guidage (17) possède un diamètre d'environ 0,28 mm (0,011 pouce) et que la lumière interne a un diamètre interne d'environ 0,33 mm (0,013 pouce).

5. Appareil selon les revendications 1, 2, 3 ou 4, caractérisé en outre en ce que le cathéter (11) possède un ballonnet gonflable (13) positionné coaxialement autour de la tige tubulaire (16) vers l'extrémité distale du cathéter (11).

6. Appareil selon la revendication 5, dans lequel l'élément tubulaire (16) s'étend au-delà de l'extrémité distale du ballonnet (13).

7. Appareil selon l'une quelconque des revendications 1 à 6, caractérisé en outre par un revêtement tubulaire (24) en un matériau antifriction qui s'étend coaxialement à l'intérieur de la partie terminale distale deladite tige tubulaire (16).

8. Appareil selon la revendication 5, caractérisé en outre par ledit cathéter (11) qui comprend un élément tubulaire externe (12) comprenant ledit ballonnet gonflable (13) ; ladite tige tubulaire qui s'étend coaxialement à l'intérieur dudit élément tubulaire externe (12), un passage étant formé entre la paroi externe de ladite tige (16) et la paroi interne de la tige tubulaire externe (12) en vue du gonflement du ballonnet ; un revêtement (24) en polytétrafluoroéthylène qui possède une ouverture luminale de diamètre relativement faible et s'étend axialement à l'intérieur de ladite tige tubulaire (16), ledit fil de guidage (17) s'étendant axialement à l'intérieur de ladite ouverture luminale sur toute sa longueur dans ladite tige tubulaire (16).

9. Appareil selon la revendication 8, caractérisé en ce que ledit revêtement (24) s'étend au-delà de l'extrémité distale du ballonnet (13).

10. Appareil selon les revendications 8 ou 9, caractérisé en outre par un tube (23) qui est plus rigide que le revêtement (24), ledit tube (23) étant disposé à l'intérieur de la partie terminale proximale de la tige tubulaire (16) et ayant une ouverture luminale ayant sensiblement le même diamètre que l'ouverture luminale dudit revêtement (24).

11. Appareil selon la revendication 10, caractérisé en outre en ce que le tube (23) est en acier inoxydable.

12. Appareil selon l'une quelconque des revendications 8 à 11, caractérisé en outre par un adaptateur à deux bras (44) relié à l'extrémité proximale du cathéter (11) et présentant un premier orifice (46) en communication de fluide avec l'ouverture luminale dans l'élément tubulaire interne et un second orifice (47) qui est en communication de fluide avec le passage annulaire (18), le premier orifice (46) ayant un passage axial (48) ayant sensiblement le même diamètre que l'ouverture luminale définie par le revêtement (24) et le tube (23), ledit passage axial étant aligné avec lesdites ouvertures.

13. Appareil selon l'une quelconque des revendications précédentes, caractérisé en ce que la partie terminale distale du fil de guidage (17) présente une pluralité de tronçons (26-28) de diamètre de plus en plus réduit, des cônes réducteurs graduels (31-33) étant intercalés entre les tronçons.

14. Appareil selon la revendication 13, caractérisé par un enroulement hélicoïdal (36) à l'extrémité distale du fil de guidage (17), ledit enroulement hélicoïdal (36) ayant un diamètre externe inférieur ou égal au diamètre de l'ouverture luminale dans ledit revêtement (24).

15. Appareil selon l'une quelconque des revendications 8 à 14, caractérisé par un élément tubulaire (21) relativement rigide, situé coaxialement à l'intérieur de la partie terminale proximale de l'élément tubulaire externe (12).

16. Appareil selon la revendication 15, caractérisé en ce que la partie terminale distale de l'élément tubulaire (21) s'effile en un diamètre de paroi plus mince vers son extrémité distale.

17. Appareil selon l'une quelconque des revendications 8 à 16, caractérisé en outre en ce que ledit élément tubulaire externe (12) est relativement souple et s'étend sur toute la longueur du cathéter de dilatation par ballonnet.

18. Assemblage de cathéter de dilatation par ballonnet selon l'une quelconque des revendications 10 à 17, caractérisé en ce que les ouvertures luminales dans ledit revêtement (24) et ledit tube (23) ont un diamètre de 0,33 mm (0,013 pouce).

19. Appareil selon l'une quelconque des revendications 4 à 18, caractérisé en outre en ce que ledit corps de cathéter (11) présentant ledit ballonnet gonflable (13) possède une première lumière interne qui est en communication de fluide avec l'intérieur du ballonnet gonflable (13) et une source de fluide gonflant et des moyens pour sceller l'extrémité distale du ballonnet (13), afin de prévenir la perte de fluide gonflant de l'intérieur de ce dernier.
